# EUROPEAN PATENT APPLICATION

(11) **EP 1 495 670 A1**
(43) Date of publication of application: **12.01.2005**
(21) Application number: 03746473.2
(22) Date of filing: 14.04.2003
(51) Int. Cl.: A01H 1/00, C12N 15/09

(54) **PLANT TRANSFORMATION SYSTEM**

(30) Priority: 15.04.2002 JP 2002111943
(71) Applicant: Phytoculture Control Co., Ltd.,, Izumisano-shi, Osaka 598-0022 (JP)
(72) Inventor: AKAI, Tatsuo, c/o PHYTOCULTURE CONTROL CO., LTD., Izumisano-shi, Osaka 598-0022 (JP); SUZUMURA, Daisuke, PHYTOCULTURE CONTROL CO., LTD., Izumisano-shi, Osaka 598-0022 (JP); KOJIMA, Tomoko, c/o PHYTOCULTURE CONTROL CO., LTD., Izumisano-shi, Osaka 598-0022 (JP); KOBAYASHI, Akio, Toyonaka-shi, Osaka 560-0012 (JP); FUKUSAKI, Eiichiro, Suita-shi, Osaka 565-0855 (JP)
(74) Representative: von Kreisler Selting Werner
(86) International application number: PCT/JP2003/004681
(87) International publication number: WO 2003/086053

(57) **Abstract**

There is provided an equipment for transforming plants which comprises: a microporous body having a surface on which a plant seed is germinated and grown into a plant body, wherein the plant seed is germinated and grown by absorbing an aqueous nutrition which is retained in communicating pores in the microporous body from the surface of the microporous body; and a carrier solution containing a gene with which the plant body is transformed, wherein the grown plant body is transformed by immersing it in the carrier solution according to an in planta method. According to the equipment for transforming plants of the present invention, a method for experimenting, investigating and developing higher plants can be conducted more exactly, conveniently, speedy and efficiently.

## Description

### FIELD OF THE INVENTION

The present invention relates to an equipment for transforming plants, a system for transforming plants and a method for transforming plants, more particularly, it relates to the equipment, system and method suitable for transforming plant bodies according to an in planta method. Furthermore, the present invention relates to a method for selecting plants harboring a heterogeneous gene from a parent transformed plant body.

### BACKGROUND OF THE INVENTION

At present, transformation of higher plants is greatly noticed, as it leads to acceleration or efficiency of breeding and improvement of plants for increase in food production, and production of useful substances inherent in plants. Among them, mouse-ear cress, rice and tobacco and the like which are particularly investigated as a model plant of higher plants are enthusiastically transformed by a convenient in planta method, and a speed for investigating or developing them is being accelerated. Therefore, a method for experimenting, investigating and developing higher plants more exactly, conveniently, speedy and efficiently is demanded, which can provide a healthy and high quality plant in a qualitatively and quantitatively stable manner.

However, in a conventional transformation of a plant body by an in planta method, environmental management such as irrigation was required, because a plant body to be transformed was grown in a compost in a vessel such as a pot for growing a plant. In addition, in the conventional method, a size of one vessel was large relative to a number or a size of the plant to be used, thereby, a larger space and a larger environment-controlling facility were required in order to grow a number of plant bodies. Accordingly, in the conventional method, an experimental efficiency could be enhanced only at a relatively large institute. In addition, in the conventional method, an experiment in which a growth degree at transformation of the plant bodies to be used is severely uniformized could not be performed, because transformation of the plant bodies was conducted in a vessel unit. In addition, there are often plant bodies not suitable for immersing into a carrier solution. From above reasons, the experimental efficiency in the conventional method was low. Moreover, there were disadvantages in the conventional method that the compost is also immersed in a solution for transformation, that impurities are mixed in the solution, that the solution is infiltrated into the compost, and the like. In addition, in the case where a vacuum infiltration transformation of the in planta method is used, the number of plants which can be treated in one operation is limited because a volume of a vacuum chamber to be used is limited. Furthermore, there were problems in the conventional method that a special space is required for an acclimatization treatment after transformation, and that management of plant bodies until flowering and fructification is likely to go wrong.

In addition, hitherto, selection of plant bodies harboring a heterogeneous gene from a parent transformed plant body has been generally conducted by determining whether a seed has an ability to germinate or grow on a selection medium containing a drug for the presence or absence of a drug-resistant gene which has been introduced simultaneously with the heterogeneous gene. Thereafter, the grown plant is repotted to the compost. However, there are problems that in the case where multiple selections are conducted, cumbersome handling is required in which the plant seed or the plant body is transferred to different selective media at every selection, and that upon repotting the plant body from the selective medium to the compost, plant roots are cut. Moreover, a conventional selection method is undesirable, because it is also thought that a stress is burden on the plant body due to a rapid environmental change upon transferring it between a plurality of selective media or from under a selection condition to under a normal growth condition.

As described above, in the conventional transformation method of the plant body, wherein the plant body is grown in a compost, and in a conventional selection method of the plant harboring the heterogeneous gene from the parent transformed plant, a method for experimenting, investigating and developing higher plants more exactly, conveniently, speedy and efficiently could not be conducted.

### SUMMARY OF THE INVENTION

The present inventors studied intensively in view of above problems and, as the result, found that above problems can be solved by growing a plant body to be transformed using a microporous body, and subjecting it as such to transformation according to an in planta method, or by germinating or growing a seed obtained from a transformed plant on the microporous body which has been immersed in a selective medium, which resulted in completion of the present invention.

That is, in the first aspect, the present invention provides an equipment for transforming plants which comprises:
a microporous body having a surface on which a plant seed is germinated and grown into a plant body, wherein the plant seed is germinated and grown by absorbing an aqueous nutrition which is retained in communicating pores in the microporous body from the surface of the microporous body; and
a carrier solution containing a gene with which the plant body is transformed,
wherein the grown plant body is transformed by immersing it in the carrier solution according to an in planta method.

According to the first aspect of the present invention, no environmental management such as irrigation is required for a long period of time because the plant body is grown by absorbing the aqueous nutrition supplied by the microporous body. In addition, according to the first aspect of the present invention, an area occupied by one plant body can be reduced, a large numbers of plant bodies can be efficiently grown and they can be efficiently subjected to transformation because soil is not required for growing or transforming the plant body. In addition, according to the first aspect of the present invention, an experiment in which only plant bodies of severely uniformized in their growth degree are selected and subjected them to transformation can be performed because the plant body can be independently grown in one unit. In addition, an exact experiment can be performed without contamination of any impurity into a solution for transformation, and the plant body can be easily handled, because no flowing medium such as soil is used in the experiment. In addition, in the conventional method, it is necessary that the carrier solution must be squeezed from the soil in light of biological containment, because the carrier solution is easily infiltrated into the soil upon transformation. Thereby, in the conventional method, an experimental or working efficiency is lowered and often a stress is burden to the plant body. On the contrary, in the present invention, microorganisms and the like in the carrier solution are hardly penetrated into the microporous body even in the case where the microporous body is contacted with the carrier solution upon transformation because the microporous body has been filled with the aqueous solution, thereby, it is not necessary that the carrier solution must be squeezed from the microporous body. Therefore, in the present invention, such the experimental or working efficiency can be enhanced, and the stress is not burden to the plant body. In addition, although the microporous body should be sterilized in a limited space such as in an autoclave and the like after use in light of biological containment and recycling or a disposition, the microporous body of the present invention can be efficiently treated even in such the case for reasons as described above.

In addition, in the second aspect, the present invention provides the equipment for transforming plants of the first aspect of the present invention, wherein the in plant method is a vacuum infiltration transformation.

According to the second aspect of the present invention, a transformation efficiency of the plant body can be further enhanced.

In addition, in the third aspect, the present invention provides a system for transforming plants which comprises:
a plurality of microporous bodies, each microporous body having a surface on which a plant seed is germinated and grown into a plant body; and
a holding means for removably holding the plurality of microporous bodies,
wherein each plant seed is germinated and grown by absorbing an aqueous nutrition retained in communicating pores in the microporous body from the surface of the microporous bodies, and
wherein a plurality of plant bodies grown on the surfaces of the microporous bodies held by the holding means are transformed by immersing them in a carrier solution approximately at the same time according to an in planta method.

According to the third aspect of the present invention, in addition to advantages of the first aspect of the present invention, a further accelerated and efficient experiment can be performed because a plurality of plant bodies can be subjected to transformation at the same time in parallel.

In the fourth aspect, the present invention provides the system for transforming plants of the third aspect of the present invention, wherein the in planta method is a vacuum infiltration transformation.

According to the fourth aspect of the present invention, the transformation efficiency of plant bodies in the third aspect of the present invention can be further enhanced.

In the fifth aspect, the present invention provides the system for transforming plants of the third or fourth aspect of the present invention, wherein the aqueous nutrition is contained in a holding means with contacting with the microporous bodies.

According to the fifth aspect of the present invention, the system may be comprised of only the microporous bodies and the holding means because the aqueous nutrition can be contained in the holding means, thereby, the system can be a more simple and convenient construction and the experiment can be performed more conveniently and efficiently and in a smaller space.

In the sixth aspect, the present invention provides the system for transforming plants of the third or fourth aspect of the present invention, which further comprises a storage tank for storing the aqueous nutrition and an aqueous nutrition-supplying means for connecting the microporous bodies with the aqueous nutrition in the storage tank, wherein the aqueous nutrition in the storage tank is supplied to the microporous bodies through the aqueous nutrition-supplying means.

According to the sixth aspect of the present invention, in addition to the advantages of the third and fourth aspects of the present invention, requirements such as a size of the microporous body and a volume of the aqueous nutrition may be loosen. Thereby, for example, the aqueous nutrition may be supplied from the storage tank through the aqueous nutrition-supplying means even in the case where the microporous body is short, the microporous body can be further miniaturized, and the experiment can be performed more conveniently.

In addition, in the seventh aspect, the present invention provides the system for transforming plants of any one of the third to sixth aspects of the present invention, wherein the microporous body has a cylindrical shape, and the plant seed is germinated and grown on an inner surface of the microporous body.

According to seventh aspect of the present invention, an area occupied by one microporous body can be further reduced, and a large numbers of the plant bodies can be efficiently grown in a smaller space, and can be efficiently subjected to the in planta method.

In addition, in the eighth aspect, the present invention provides the system for transforming plants of any one of the third to seventh aspects of the present invention, wherein the plants are selected from the group consisting of a useful tree such as bishop's flower (Ammi majus), onion (Allium cepa), garlic (Allium sativum), celery (Apium graveolens), asparagus (Asparagus officinalis), sugar beet (Beta vulgaris), cauliflower (Brassica oleracea var. botrytis), brusseles sprout (Brassica oleracea var. gemmifera), cabbage (Brassica oleracea var. capitata), rape (Brassica napus), caraway (Carum carvi), chrysanthemum (Chrysanthemum morifolium), spotted hemlock (Conium maculatum), coptis Rhizome (Coptis japonica), chicory (Cichorium intybus), summer squash (Curcurbita pepo), thorn apple (Datura meteloides), carrot (Daucus carota), carnation (Dianthus caryophyllus), buckwheat (Fagopyrum esculentum), fennel (Foeniculum vulgare), strawberry (Fragaria chiloensis), soybean (Glycine max), hyacinth (Hyacinthus orientalis), sweet potato (Ipomoea batatas), lettuce (Lactuca sativa), birds-foot trefoil (Lotus comiculatus, Lotus japonicus), tomato (Lycopersicon esculentum), alfalfa (Medicago sativa), tobacco (Nicotiana tabacum), rice (Oryza sativa), parsley (Petroselinum hortense), pea (Pisum sativum), rose (Rosa hybrida), egg plant (Solanum melongena), potato (Solanum tuberosum), wheat (Triticum aestivum), maize (Zea mays), sugar beat(Beta vulgaris), cotton (Gossypium indicum), rape (Brassica campestris), flax (Linum usitatissimum), sugarcane (Saccharum officinarum), papaya (Carica papaya), Squash (Cucurbita moschata), cucumber (Cucumis sativus), watermelon (Citrullus vulgaris), melon (Cucumis melo), Winter Squash (Cucurbita maxima) and the like; a foliage plant such as snapdragon (Antirrhinum majus), mouse-ear cress (Arabidopsis thaliana), croton (Codiaeum variegatum), cyclamen (Cyclamen persicum), poinsettia (Euphorbia pulcherrima), barberton daisy (Gerbera jamesonii), sunflower (Helianthus annuus), fish geranium (Pelargonium hortorum), petunia (Petunia hybrida), African violet (Saintpaulia ionatha), dandelion (Taraxacum officinale), torenia (Torenia fournieri), Dutch clover (Trifolium repens), cymbidium (Cymbidium) and the like; a woody plant such as beat tree (Azadirachta indica), orange (Citrus), common coffee (Coffea arabica), ribbon gum (Eucalyptus), para rubber tree (Hevea brasiliensis), holly (Ilex aquifolium), trifoliate orange (Poncirus trifoliata), almond (Prunus amygdalus), carolina poplar (Populus canadensis), oriental arborvitae (Biota orientalis), Japanese ceder (Cryptomeria japonica), Norway spruce (Picea abies), pine genus (Pinus), grapevine (Vitis vinifera), apple (Malus pumila), apricot (Prunus armeniaca), persimmon (Diospyros kaki), fig (Ficus carica), chestnut (Castanea crenata), Lombardy poplar(Populus nigra), Eleuthero(Acanthopanax senticosus) and the like.

According to the eighth aspect of the present invention, an experiment can be performed more rapidly and efficiently for plant bodies on which investigation utilizing transformation is enthusiastically performed at present.

In addition, in the ninth aspect, the present invention provides a method for transforming plants which comprises steps of:
germinating and growing a plant seed into a plant body on a surface of a microporous body, wherein the plant seed is grown by absorbing an aqueous nutrition retained in communicating pores in the microporous body from the surface of the microporous body; and
transforming the plant body grown on the surface of the microporous body by immersing it in a carrier solution containing a gene with which the plant body is transformed according to an in planta method.

According to the ninth aspect of the present invention, there can be provided a method for transforming plants having advantages as described for the first aspect of the present invention.

In addition, in the tenth aspect, the present invention provides the method for transforming plants of the ninth aspect of the present invention, wherein the in planta method is a vacuum infiltration transformation.

According to the tenth aspect of the present invention, there can be provided a method for transforming plants having advantages as described for the second aspect of the present invention.

In addition, in the eleventh aspect, the present invention provides a method for transforming plants which comprises steps of:
removably holding a plurality of microporous bodies in a holding means;
seeding a plant seed on each surface of the microporous bodies, wherein the plant seed is germinated and grown into a plant body by absorbing an aqueous nutrition retained in communicating pores in the microporous body from the surface of the microporous body; and
transforming a plurality of plant bodies grown on the surfaces of a plurality of the microporous bodies held in the holding means by immersing them in a carrier solution containing a gene with which the plant body is transformed approximately at the same time according to an in planta method.

According to the eleventh aspect of the present invention, there can be provided a method for transforming plants having advantages as described for the third aspect of the present invention.

In addition, in the twelfth aspect, the present invention provides the method for transforming plants of eleventh aspect of the present invention, wherein the in planta method is a vacuum infiltration transformation.

According to the twelfth aspect of the present invention, there can be provided a method for transforming plants having advantages as described for the fourth aspect of the present invention.

In addition, in the thirteenth aspect, the present invention provides the method for transforming plants of the eleventh or twelfth aspect of the present invention, which further comprises a step of selecting only microporous bodies having the plant bodies which have grown to a stage suitable for transformation to hold them in the holding means before immersing the plant bodies in the carrier solution, and subjecting the plant bodies to transformation.

According to the thirteenth aspect of the present invention, only plant bodies exactly uniformized in their growth stage can be subjected to the transformation experiment, thereby, a more exact experiment can be conducted.

In addition, in the fourteenth aspect, the present invention provides a method for selecting plants harboring a heterogeneous gene from a parent transformed plant body, which comprises steps of:
(i) immersing a portion of a microporous body in an aqueous nutrition containing one or more of the first drug for selection;
(ii) seeding a plant seed obtained from a transformed plant body on a surface of the microporous body, wherein the transformed plant body has been transformed with at least one heterogeneous gene comprising a resistant gene for the first drug for selection and wherein the plant seed harboring the heterogeneous gene from a parent transformed plant body can be germinated or grown by absorbing an aqueous nutrition containing the first drug for selection retained in communicating pores in the microporous body from the surface of the microporous body, but the plant seed harboring no heterogeneous gene from a parent transformed plant body can not be germinated or grown; and
(iii) obtaining the plant body which can be germinated or grown or, further, repeating above steps (i) to (iii) once or more times, using the plant seed obtained from the plant body and one or more of the drug for selection which is same as or different from the first drug for selection in place thereof, wherein the transformed plant body also comprises a resistant gene for the drug for selection.

According to the fourteenth aspect of the present invention, the plant body harboring the heterogeneous gene can be conveniently subjected to a plurality of selections by merely changing the aqueous nutrition for selection into which the microporous body is immersed. In addition, a root of a grown plant body is not cut because there is no necessity that the plant body is repotted to the compost. In addition, in the case where subjecting to different selections, a stress due to a rapid environmental change is not burden on the plant seed or the plant body because the aqueous nutrition for selection is gradually changed in the microporous body. Thereby, the plant body harboring the heterogeneous gene from the parent transformed plant body and the plant seed obtained from the plant body can be selected in more exactly, conveniently, speedy and efficiently.

Furthermore, in the fifteenth aspect, the present invention provides a method for selecting plants harboring a heterogeneous gene from a parent transformed plant body which comprises conducting, at least once, steps of:
seeding a plant seed obtained in the fourteenth aspect harboring the resistant gene for the first drug for selection on the surface of the microporous body, wherein the plant seed is germinated and grown into a plant body by absorbing one or more of a drug for selection different from the first drug for selection or the aqueous nutrition retained in communicating pores in the microporous body from the surface of the microporous body; and
confirming whether the grown plant body harbors the resistant gene for the drug for selection different from the first drug for selection, or whether the grown plant body expresses a target heterogeneous gene as a phenotype thereof.

According to the fifteenth aspect of the present invention, in addition to advantages as described for the fourteenth aspect of the present invention, the plant harboring the heterogeneous gene from the parent transformed plant body and expressing it as phenotype can be selected more exactly.

The term "plant seed" herein means a plant seed in a state which has not been germinated yet. In addition, the term "plant body " herein means a plant from a plant after germination to a plant grown to the suitable stage for transformation according to the in plant method. And, in the case where it is simply referred to as "plant" herein, it means that both of such the plant seed and plant body are included. In addition, the term "transformed plant body" herein means a plant body to which a heterogeneous gene is introduced by the method of the present invention or other method for transformation well known in the art, and the term "heterogeneous gene" means any extraneous gene for being intentionally introduced into the plant, including a gene participating in plant morphogenesis, a gene of particular enzyme, a gene participating in production of a useful material, a gene relating to disease resistance and the like, in addition to a marker gene of a drug resistance for screening the transformed plant.

The term "carrier solution" herein means a suspension or a homogenate of a carrier such as bacteria and virus which carries a plasmid vector comprising a particular heterogeneous gene, with which the plant body can be transformed according to the in plant method. Examples of the carrier solution include, for example, a suspension of Agrobacterium tumefaciens, Agrobacterium rhizogenes and the like, and an additive suitable for transformation of the plant body by the in planta method, for example, a buffer, an osmoregulating agent, a pH-adjusting agent, a surface active agent, a plant growth-adjusting agent and the like can be contained therein. In addition, for the method for transforming, the transformation efficiency can be further enhanced by utilizing a vacuum infiltration transformation among the in planta method, and even in that case, similar additives can be contained in the carrier solution and similar carriers can be used.

In addition, in the case where the terms "upper end" and "lower end" are used herein, they mean a side of the microporous body to which the plant seed is placed and an opposite side thereto, respectively.

### BRIEF DESCRIPTION OF THE DRAWINGS

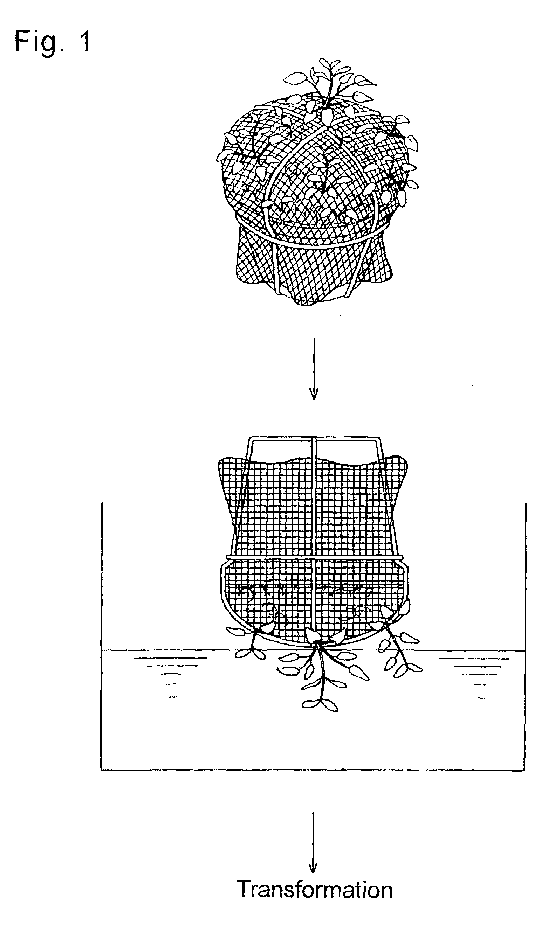

Figure 1 shows a method for transforming a plant body according to a conventional in planta method.

Figure 2 shows a system for transforming plants of an embodiment of the present invention.

Figure 3 shows a system of an embodiment of the present invention, in which a plurality of microporous bodies fitted in one holding means are placed in a storage tank.

Figure 4 is a partial cross section view showing a microporous body provided with an aqueous nutrition-supplying means.

Figure 5 shows a system for transforming plants of an embodiment of the present invention in which a plurality of microporous bodies are fitted into a holding means having tapered recessions.

Figure 6 shows steps for transforming plants using the system for transforming plants of the embodiment shown in Figure 5 according to the in planta method.

Figure 7 shows transformation of plants with a system for transforming plants of an embodi ment of the present invention while it is floated on a carrier solution according to the in planta method.

Figure 8 shows a system for transforming plants of an embodiment of the present invention.

Figure 9 shows the system for transforming plants shown in Figure 8 and a carrier solution tank into which the system is fitted to immerse the plant body in a carrier solution.

Figure 10 shows caps equipped with a hook or a magnet for suspending the microporous body from the holding means.

Figure 11 shows a system for transforming plants of an embodiment of the present invention.

Figure 12 shows a system for transforming plants of an embodiment of the present invention.

Figure 13 shows a system for transforming plants of an embodiment of the present invention.

Figure 14 shows a storage tank of an embodiment of the present invention.

Figure 15 shows a system for transforming plants of an embodiment of the present invention.

### DETAILED DESCRIPTION OF THE INVENTION

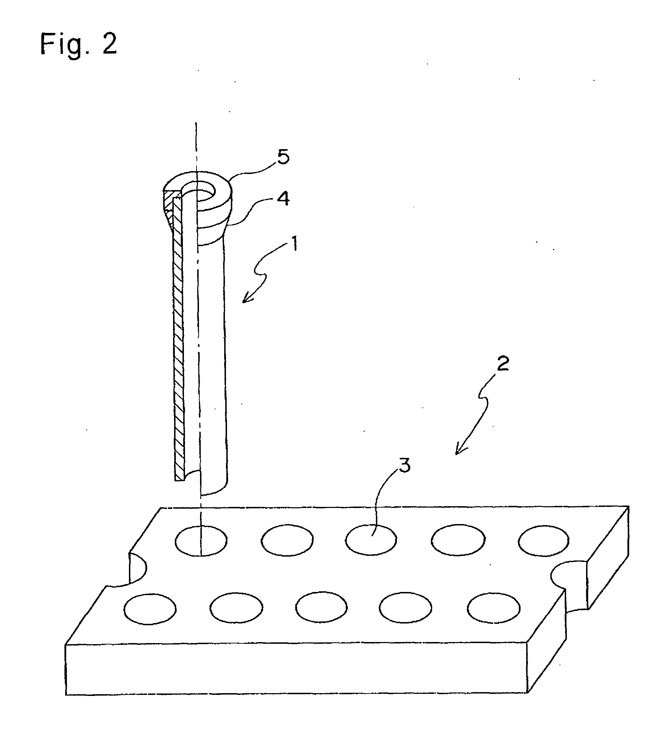

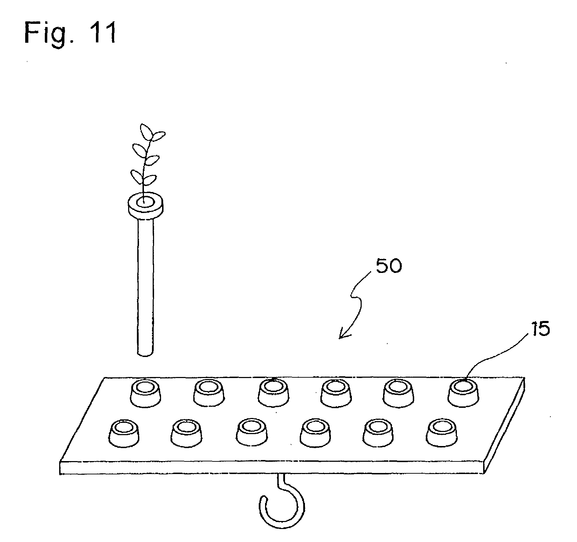

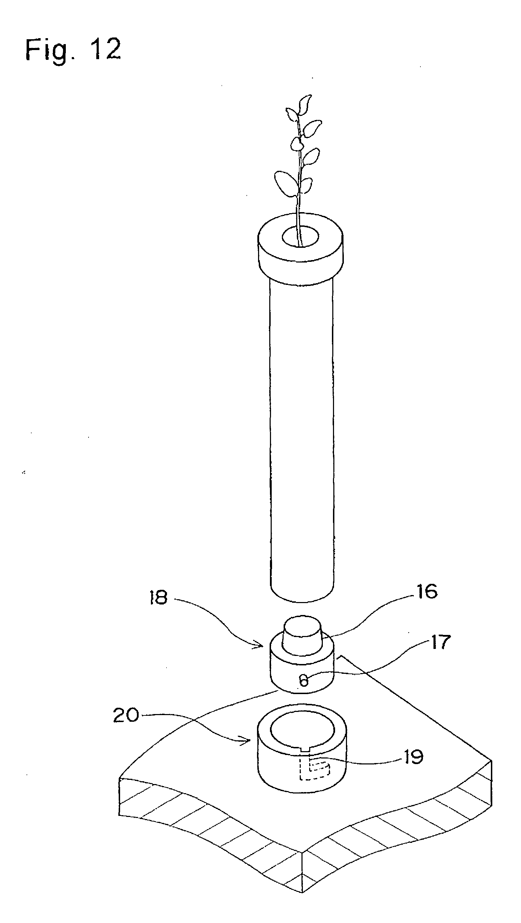

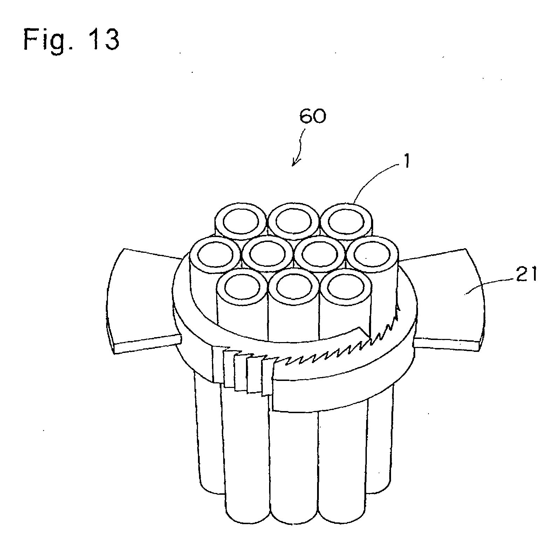

Next, embodiments of the present invention will be illustrated with referring to Figures 2-15. Herein, a system for transforming plants of the present invention will be mainly illustrated together with an equipment for transforming plants, a method for transforming plants and a method for selecting plants, because the equipment for transforming plants of the present invention corresponds to a microporous body and a carrier solution used in the system for transforming plants of the present invention.

Firstly, a microporous body (1) having a surface on which a plant seed is germinated and grown is shown in Figure 2, wherein the microporous body is shown in the form of a cylindrical shape. After the microporous body is adequately supplied with an aqueous nutrition and the like, the plant seed is seeded on an inner surface of the microporous body in the vicinity of an end to germinate and grown. The interior of the microporous body comprises communicating pores and, in the case where the aqueous nutrition is supplied to a portion of the microporous body, it is supplied throughout the microporous body through the communicating pores to be retained inside the microporous body. The seed seeded on the surface of the microporous body is germinated and grown by absorbing the aqueous nutrition which is retained inside the microporous body from the surface of the microporous body.

That is, it is not always restricted by this theory, it seems that a movement of the aqueous nutrition is caused based on a difference in suction at interfaces between the aqueous nutrition and the microporous body and betwe en the microporous body and the plant body. The aqueous nutrition retained by the microporous body is dried depending on a portion of the aqueous nutrition utilized by the plant body on the microporous body, thereby, a force due to the capillary action is restored depending on the dried portion of the aqueous nutrition in the microporous body. As the result, the microporous body absorbs and retains the aqueous nutrition again, and only a necessary amount of the aqueous nutrition is always supplied to the plant. Therefore, according to the present invention, even a plant susceptible to dry or high wet environment can be stably and conveniently germinated and grown.

In addition, a holding means (2) which can removably hold a plurality of microporous bodies (1) is shown in Figure 2, wherein a pore having an inner diameter which is approximately equal to an outer diameter of the microporous body (3) is provided in the holding means (2) such that the cylindrical microporous body can be removably held by the holding means. On the other hand, a taper (4) and a ring (5) are provided at one end of the microporous body. These taper (4) and ring (5) ensure that the microporous body (1) can be fitted into the pore (3) to hold it at a predetermined position even where the outer diameter of the microporous body and the diameter of the holding means are not produced at a high dimensional accuracy. In addition, after fitting the microporous body into the holding means, another ring which is not shown in figures may be run from a lower end of the microporous body at the lower side of the holding means (2) to sandwich the holding means (2). Thereby, the microporous body (1) can be securely held in the holding means (2) while a position of the microporous body (1) in the holding means (2) is not slipped even when they are inverted. Such the taper and the ring may be made of a material identical to that of the microporous body (1), or may be made of a material such as resin or the like. Alternatively, for the taper (4) and the ring (5), the microporous body itself may be one-piece molded so as to have shapes of the taper and the ring. The system for transforming plants of the present invention comprises at least the microporous body and the holding means as described above.

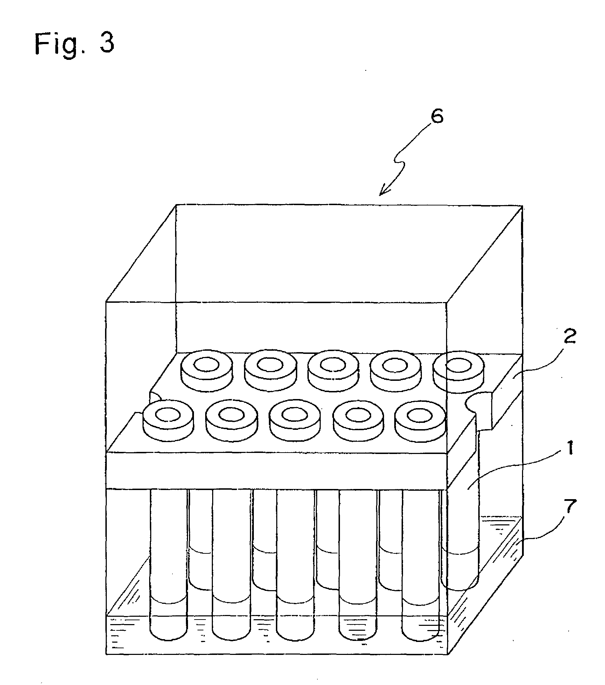

Next, as shown in Figure 3, portions of a plurality of microporous bodies which have been fitted into the pores (3) of the holding means (2) as described above are immersed in the aqueous nutrition (7) stored in the storage tank (6). After the aqueous nutrition is supplied throughout the microporous body, the plant seed is seeded on the inner surface of the microporous body (1) in the vicinity of the upper end thereof to germinate and grow by incubating under the standard germination or growth condition. In this embodiment, the microporous body is stably set in the holding means by approximately matching the size of the holding means (2) with the size of the storage tank (6).

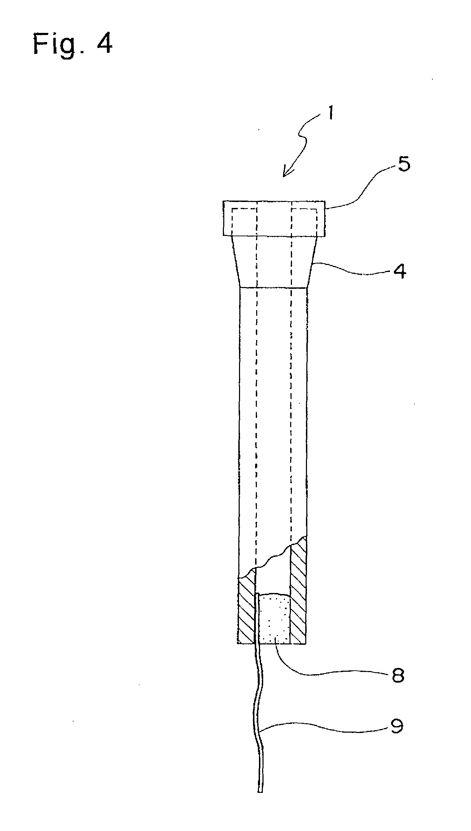

In addition, in another embodiment, as shown in Figure 4, the microporous body (1) may be provided with an aqueous nutrition-supplying means (9) and an engaging means (8) to indirectly supply the aqueous nutrition to the microporous body through the aqueous nutrition-supplying means. Such the engaging means (8) is preferably made of a material having a contraction and repulsive property such as an expanded polystyrene, an expanded polyurethane, an expanded polyethylene and the like, or the material such as of polypropylene, polyethylene and the like. In addition, the engaging means (8) is preferably made of a material and has a structure, such that it can engage the aqueous nutrition-supplying means against the microporous body without lowering an ability to supply the aqueous nutrition of the aqueous nutrition-supplying means. In addition, the aqueous nutrition-supplying means (9) is made of a material and has a shape, such that it can supply the aqueous nutrition to the microporous body even in the case where it is perpendicularly suspended from the microporous body and immersed in the aqueous nutrition at another end, and preferably is made of an open-cell type expanded plastic such as polyvinyl alcohol having a stringy shape, or a filamentous bundle or nonwoven fabric such as of glass fiber, carbon fiber, acrylic fiber and the like.

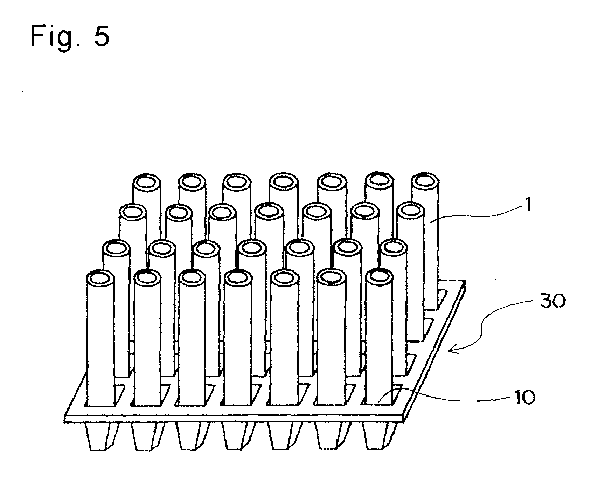

In addition, in another embodiment, in the system for transforming plants shown in Figure 5, a plurality of microporous bodies held in the holding means (30) which can removably hold the microporous body by pressing a lower end of the microporous body thereinto are shown, wherein a tapered recession (10) is provided in the holding means, and the microporous body (1) is held by pressing one end thereof thereinto. In addition, the aqueous nutrition can be directly supplied to the microporous body by storing the aqueous nutrition in the tapered recession (10) of the holding means (30), thereby, the system for transforming plants may be constructed only of the microporous body (1) and the holding means (30). Alternatively, the holding means in which a bottom portion of the tapered recession is opened, and which holds the microporous body, can be immersed in or floated on the storage tank, which is not shown in Figures, in which a predetermined amount of the aqueous nutrition is stored.

Then, a series of steps of seeding the plant seed to germinate, selecting the plant body grown to the stage suitable for transformation, and subjecting the plant body to transformation by the in plant a method according to the embodiments as described above will be illustrated below.

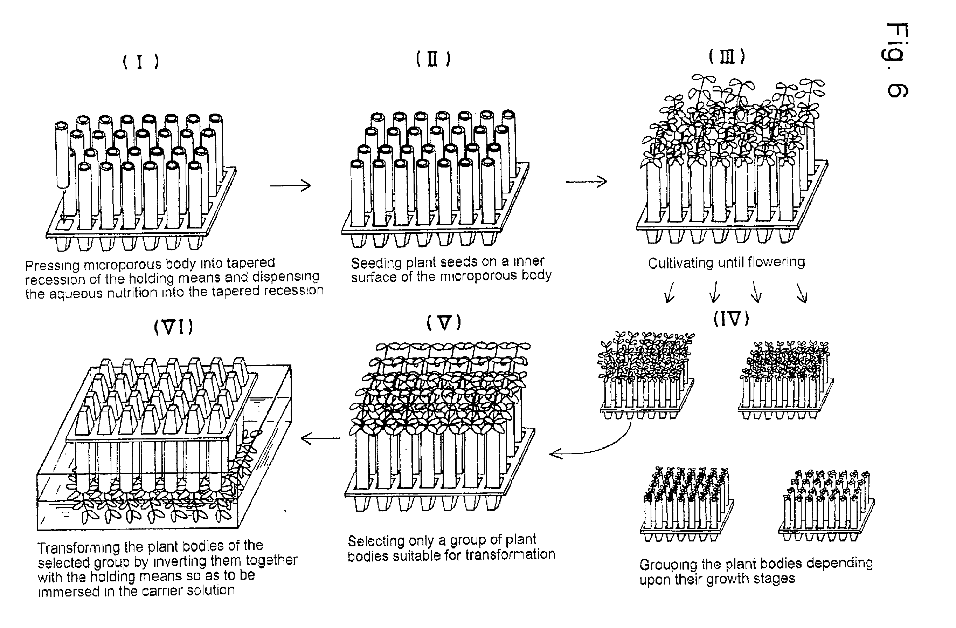

A series of steps of plant transformation shown in Figure 6 comprise a step of removably pressing a plurality of microporous bodies into the holding means and dispensing the aqueous nutrition into the tapered recession of the holding means (I), a step of seeding the plant seed on a surface of each of the microporous bodies throughout which the aqueous nutrition has been supplied (II), a step of germinating and growing the plant seed (III), a step of grouping the plant bodies depending upon their growth stages after a predetermined period of time (IV), a step of selecting only a group of plant bodies suitable for transformation (V), and a step of transforming the plant bodies of the selected group by inverting them together with the holding means so as to be immersed in the carrier solution (VI). Although an embodiment in which the holding means has the tapered recession as described above is shown here, the holding means may have any shape as far as it can hold a plurality of microporous bodies.

Furthermore, the transformed plant body can be confirmed by any method known to those skilled in the genetic engineering art, but preferably it can be confirmed by integrating a certain drug resistant marker in a carrier in advance, and then selecting a seed obtained from the plant body for an ability to grow on a medium containing the drug. Thereafter, an expression of a target heterogeneous gene aimed at introducing into the plant (for example, morphology of the plant, enzyme activity, production of particular material, disease resistance or the like) can be finally confirmed by mating the selected plants, collecting a seed therefrom, germinating and growing the seed, and selecting them.

The microporous body used in the present invention is not particularly limited as far as the aqueous nutrition which is contact with a portion thereof can be supplied and retained throughout the interior thereof through communicating pores therein, but the microporous body having a water-absorbing ability being capable of retaining usually 0.05-0.5 (wt/wt), preferably 0.05-0.3, and more preferably 0.1-0.2-fold amount of water at 20 °C, and having communicating pores having a pore diameter of usually 0.2-900 µm, preferably 0.2-80 µm, more preferably 0.2-9 µm, most preferably 0.2-3 µm at a pore rate (vol/vol) of usually 0.05-1, preferably 0.1-0.4, more preferably 0.2-0.3 relative to the microporous body is preferred.

Examples of such the microporous body include those obtained by kneading, forming and firing non-metal inorganic solid raw materials such as No.10 clay, porcelain No.2 clay (Shiroyama Cerapot Kabushiki-Kaisya) and Murakami clay (produced in Niigata Prefecture in Japan) according to the conventional method, as well as open-cell type plastic foam materials such as polyvinyl alcohol foam, polyurethane foam, polystyrene foam, vinyl chloride resin foam, polyethylene foam, polypropylene foam, phenol resin foam, urea resin foam and the like. In particular, in the case where non-metal inorganic solid raw materials are made into the microporous body which easily absorbs and releases water, it is preferable that those raw materials are fired while containing, for example, petalite or alumina at 50-60 % by weight. Generally, the petalite as described above preferably contains usually 70-90 % by weight, preferably 75-85 % by weight, more preferably 75-80 % by weight of SiO₂, usually 10-20 % by weight, preferably 12-18 % by weight, more preferably 15-17 % by weight of Al₂O₃, usually 2-5 % by weight, preferably 3-4.5 % by weight, more preferably 3.5-4.2 % by weight of LiO₂, usually 0.1-0.5 % by weight, preferably 0.2-0.5 % by weight, more preferably 0.3-0.45 % by weight of K₂O, and usually 0.5-2 % by weight, preferably 0.7-1.8 % by weight, more preferably 0.8-1.6 % by weight of inevitable impurities. In addition, non-metal inorganic raw materials may contain powdery inorganic foam. Further, the microporous body used in the present invention may be comprised of a non-metal inorganic material, the strength of which is not substantially lowered even when it absorbs water.

As a method of forming a non-metal inorganic solid raw material into the microporous body, there are forming methods which are known in the art such as slip casting forming, extruding forming, press forming and potter's wheel forming. In particular, from a viewpoint of large scale production and reduction in the cost, extruding forming is preferable. In addition, drying after forming can be performed using the conventional methods and conditions known in the art. Subsequent firing of a formed body is not particularly limited as far as firing is performed by the conventional conditions and methods. For example, oxidative firing by which a desired pore is easily obtained can be selected. A firing temperature is 1000 °C to 2000 °C, preferably 1100 °C to 1500 °C, more preferably 1150 °C to 1300 °C, and most preferably approximately 1200 °C. When a temperature for firing a non-metal inorganic solid raw material is below 1000 °C, a sulfur component easily remains and, on the other hand, when the temperature is higher than 2000 °C, the microporous body having the desired water-absorbing property can not be obtained.

On the other hand, as a method of molding a microporous body made of an open-cell type plastic foam, for example, there are melt foaming molding, solid phase foaming molding, casting foaming molding and the like.

Main steps in melt foaming molding comprise melting and kneading, molding of an unfoamed sheet, heat foaming or extrusion foaming, cooling, cutting and processing. h solid phase foaming molding, a polymer is foamed in the solid phase or in the state near the solid phase. In addition, in casting foaming molding, a liquid raw material (monomer or oligomer) is cast and foamed while reacting it in the air. In order to foam an open-cell type plastic foam, a foaming agent is generally used.

In addition, the microporous body may be any shape depending upon a kind of a subject plant and a state upon use, but preferably it may be a shape such as cylindrical shape, plate shape, pillar shape, barrel shape having a bottom, pillar shape having a honeycomb cross section, and barrel shape having a polygonal cross section (for example, hexagonal or tetragonal barrel).

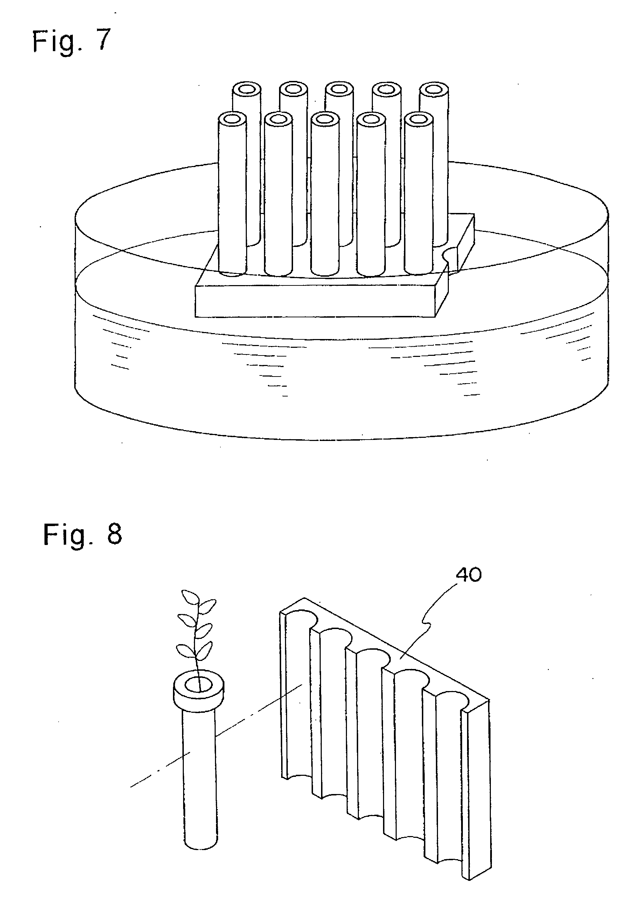

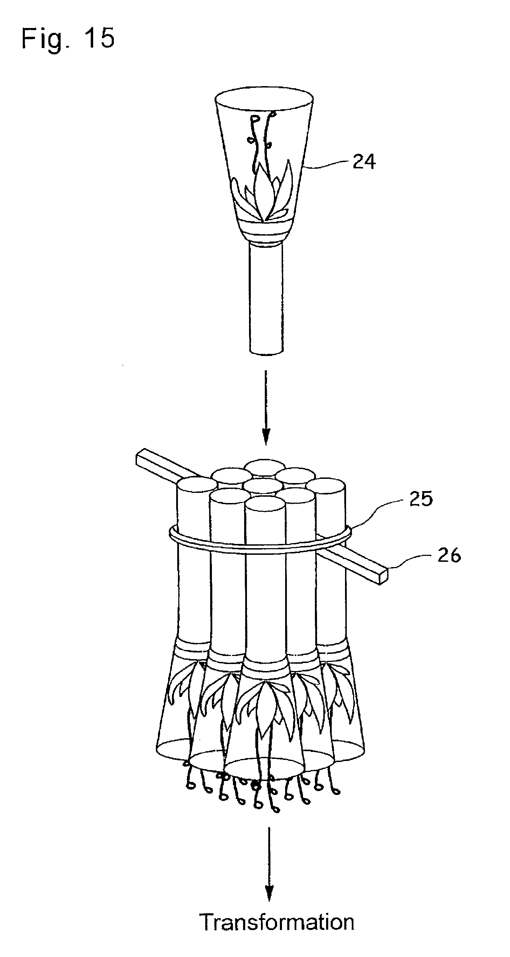

Next, the holding means used in the present invention may be made of any material and has any shape as far as it can hold the microporous body by fitting or pressing to immerse the plant body in the carrier solution and transform it by the in planta method as described above, but preferably it is made of expanded polystyrene, expanded polyethylene or expanded polyurethane of a plate shape having pores or tapered recessions as described above. In addition, the holding means is preferably made of a material having an elastic property such that the microporous body can be removably held repetitively. In addition, the holding means preferably has a specific density such that it floats on the carrier solution while the plant body is immersed in the carrier solution by inverting it as shown in Figure 7. By using the holding means having such the property, the plant body can be subjected to the in planta method by merely floating it on the carrier solution to immerse the plant body in the carrier solution without a necessity of holding the system for transforming plants of the present invention by another means. Alternatively, as shown in Figure 15, a plurality of microporous bodies may be mounted on a side wall of the storage tank by fixedly bundling them with a string or the like (25), and penetrating a stick holding means (26) between the microporous bodies.

However, in the system for transforming plants of the present invention, it is also intended that the holding mean for holding the microporous body is moved to above the carrier solution, inverted there, and put down to be immersed in the carrier solution by another transporting means which is not shown in Figures.

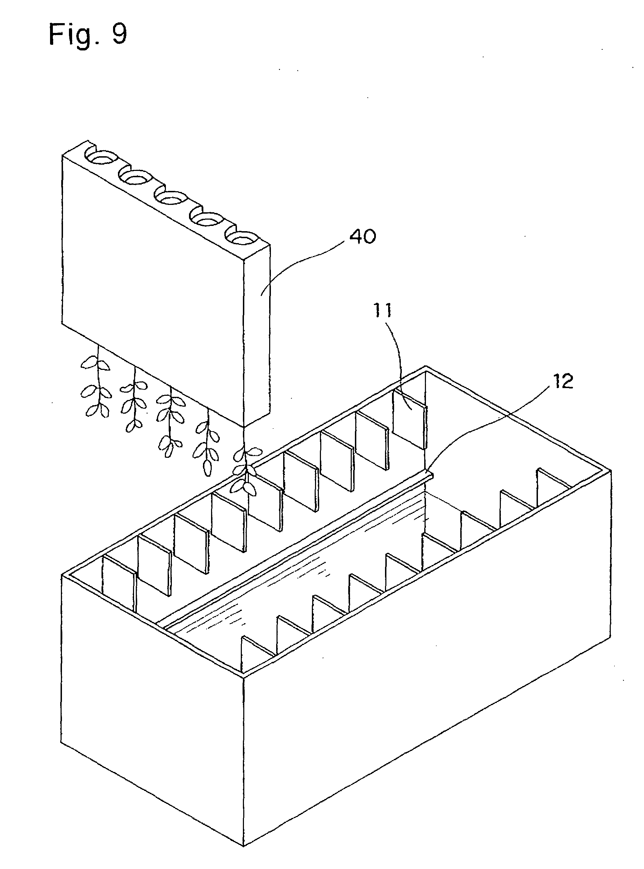

In addition, in an embodiment, the holding means used in the present invention may be one having a partial cylindrical recession (40) to which the microporous body can be attached from a side direction thereof as shown in Figure 8. In this embodiment, a plurality of plant bodies can be immersed in the carrier solution approximately at the same time by inserting the holding means, to which a plurality of microporous bodies are attached, to a tank which has a guide plate (11) and an engaging portion (12) and which contains a carrier solution (carrier solution tank) as shown in Figure 9 after the holding means is inverted.

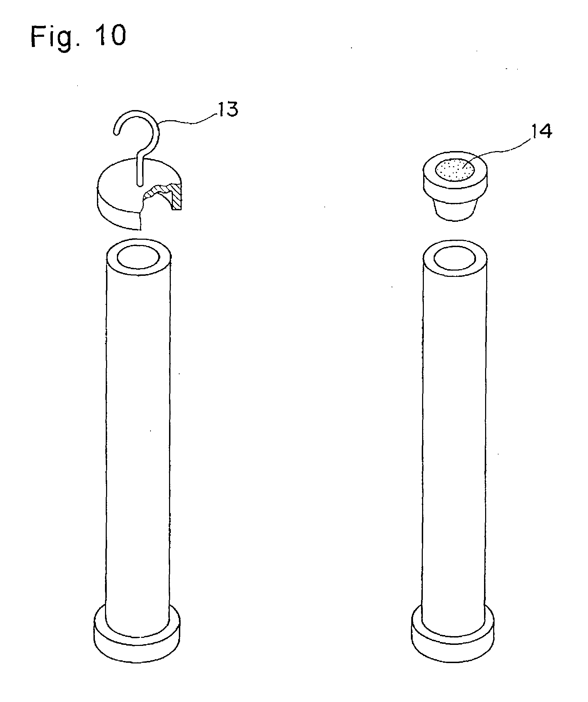

In another embodiment, the holding means used in the present invention may be one which can suspend a plurality of microporous bodies thorough caps equipped with a hook (13) or a magnet (14) as shown in Figure 10.

In addition, in another embodiment, the holding means used in the present invention may be one of a plate shape having a surface on which a recessed portion (15) having a diameter approximately same as an outer diameter of the microporous body is provided (50). In this embodiment, a plurality of microporous bodies can be held by fitting the lower end of the microporous body into this recessed portion. In addition, the plant bodies on a plurality of microporous bodies can be immersed in the carrier solution approximately at the same time by suspending the holding means by which a plurality of microporous bodies are held with a hook which is provided on an opposite side of the holding means.

In addition, in another embodiment, the holding means used in the present invention is comprised of a cap (18) having a microporous body-fitting portion (16) and a pin (17), and a plate on which a recessed portion (20) having a guide groove (19) is projected, wherein the microporous body can be stably held by guiding the pin to the groove to fit it with the cap. In this embodiment, the holding means which holds a plurality of microporous bodies can be suspended with a hook, which is not shown in Figures, provided on the opposite side of the holding means. In light of a distribution or transportation system, preferably the hook is removable.

In addition, in another embodiment, the holding means used in the present invention may be one by which side portions of a plurality of microporous bodies are fixedly supported (60). In this embodiment, a wave-shape pawl is provided in the holding means, which can fixedly support the microporous bodies such that they do not slip down from the holding means. In addition, in the holding means of this embodiment, a wing portion (21) is provided such that a labor for supporting microporous bodies with the holding means can be relieved and the microporous bodies fixedly supported by the holding means can be mounted on a side wall of the storage tank upon immersing the plant bodies in the carrier solution.

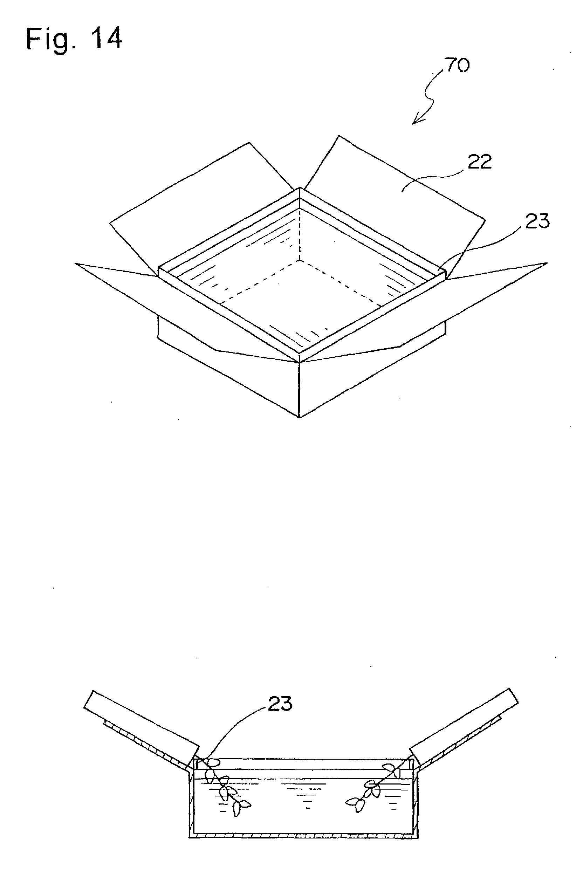

In addition, in the present invention, in the case where the plant body is immersed in the carrier solution containing the gene with which the plant body is transformed, the microporous body on which the plant body is germinated and grown in the embodiment as described above may be removed from the holding means, and it may be subjected to transformation by the in planta method by mounting on a slope (22) of a carrier solution tank (70) as shown in Figure 14. A stopping plate (23) is provided on an end of the slope of the carrier solution tank such that the plant body can be immersed in the carrier solution while the microporous body mounted on the slope is stopped at a particular position.

In addition, in the present invention, the plant body grown on the microporous body to the stage suitable for transformation by the in planta method may be surrounded with a sleeve (24) as shown in Figure 15. Thereby, even in the case where a plurality of microporous bodies and plants are densely placed, a single microporous body and plant body among them can be easily handled. In addition, the plant body can be prevented from being injured due to contact with an adjoining plant body. In addition, although it is assumed that leaves of adjoining plant bodies are adhered together, or adjoining plant bodies are injured together due to vibration upon transportation, these can be prevented by surrounding the plant body with the sleeve.

The sleeve may have any shape and may be made of any material as far as it can surround the plant body, but preferably it is comprised of a transparent material such that the condition of the plant body can be observed, and more preferably it is comprised of a plastic film such as a cellophane film. In addition, the sleeve may be fixed to the holding means in addition to an upper portion of the microporous body as shown in Figure 15 with a string, a rubber band or the like.

Examples of the plant as a subject of the system for transforming plants of the present invention are not particularly limited as far as it can be transformed by the in planta method or the vacuum infiltration transformation, but preferably include bishop's flower (Ammi majus), onion (Allium cepa), garlic (Allium sativum), celery (Apium graveolens), asparagus (Asparagus officinalis), sugar beet (Beta vulgaris), cauliflower (Brassica oleracea var. botrytis), brusseles sprout (Brassica oleracea var. gemmifera), cabbage (Brassica oleracea var. capitata), rape (Brassica napus), caraway (Carum carvi), chrysanthemum (Chrysanthemum morifolium), spotted hemlock (Conium maculatum), coptis Rhizome (Coptis japonica), chicory (Cichorium intybus), summer squash (Curcurbita pepo), thorn apple (Datura meteloides), carrot (Daucus carota), carnation (Dianthus caryophyllus), buckwheat (Fagopyrum esculentum), fennel (Foeniculum vulgare), strawberry (Fragaria chiloensis), soybean (Glycine max), hyacinth (Hyacinthus orientalis), sweet potato (Ipomoea batatas), lettuce (Lactuca sativa), birds-foot trefoil (Lotus comiculatus, Lotus japonicus), tomato (Lycopersicon esculentum), alfalfa (Medicago sativa), tobacco (Nicotiana tabacum), rice (Oryza sativa), parsley (Petroselinum hortense), pea (Pisum sativum), rose (Rosa hybrida), egg plant (Solanum melongena), potato (Solanum tuberosum), wheat (Triticum aestivum), maize (Zea mays), sugar beat(Beta vulgaris), cotton (Gossypium indicum), rape (Brassica campestris), flax (Linum usitatissimum), sugarcane (Saccharum officinarum), papaya (Carica papaya), Squash (Cucurbita moschata), cucumber (Cucumis sativus), watermelon (Citrullus vulgaris), melon (Cucumis melo), Winter Squash (Cucurbita maxima) and the like; a foliage plant such as snapdragon (Antirrhinum majus), mouse-ear cress (Arabidopsis thaliana), croton (Codiaeum variegatum), cyclamen (Cyclamen persicum), poinsettia (Euphorbia pulcherrima), barberton daisy (Gerbera jamesonii), sunflower (Helianthus annuus), fish geranium (Pelargonium hortorum), petunia (Petunia hybrida), African violet (Saintpaulia ionatha), dandelion (Taraxacum officinale), torenia (Torenia fournieri), Dutch clover (Trifolium repens), cymbidium (Cymbidium) and the like; a woody plant such as beat tree (Azadirachta indica), orange (Citrus), common coffee (Coffea arabica), ribbon gum (Eucalyptus), para rubber tree (Hevea brasiliensis), holly (Ilex aquifolium), trifoliate orange (Poncirus trifoliata), almond (Prunus amygdalus), carolina poplar (Populus canadensis), oriental arborvitae (Biota orientalis), Japanese ceder (Cryptomeria japonica), Norway spruce (Picea abies), pine genus (Pinus), grapevine (Vitis vinifera), apple (Malus pumila), apricot (Prunus armeniaca), persimmon (Diospyros kaki), fig (Ficus carica), chestnut (Castanea crenata), Lombardy poplar(Populus nigra), Eleuthero(Acanthopanax senticosus) and the like.

In addition, the aqueous nutrition used in the present invention is an aqueous solution which contains an inorganic element such as nitrate nitrogen, ammonium nitrogen, phosphorus, potassium, calcium, magnesium, iron and manganese, copper, zinc, molybdenum, boron and the like; all kinds of vitamins such as thiamin, pyridoxine, nicotinic acid, biotin, folic acid and the like; a natural material such as coconut milk, casein hydrolysate, yeast extract and the like; an organic nitrogen source such as glutamic acid, aspartic acid, alanine and the like; an agent for controlling plant growth such as auxin, cytokine, gibberellin and the like; a carbon source such as dextrose, sucrose, fructose, maltose and the like; an antibiotic such as kanamycin, hygromycin and the like; and an agrochemical such as basta and the like.

In addition, the in planta method and the vacuum infiltration transformation can be performed, for example, as described in Bechtold N, Ellis J, Pelletier (1993) "In planta Agrobacterium mediated gene transfer by infiltration of adult Arabidopsis thaliana plants." C. R. Acad. Sci. Paris, Life Sciences 316:1194-1199. or "A Model Plant Laboratory Manual" ed. M. Iwabuchi, K. Okada, and I. Shimamoto, Springer-Verlag Tokyo, 2000, April 13. Briefly, in the vacuum infiltration transformation, for example, a shape of the plant body grown on the microporous body of the present invention is conditioned such that it becomes suitable for transformation, and then the plant body is immersed in the carrier solution for transformation. The plant body is placed in a vacuum chamber while immersing in the carrier solution. The plant body is placed under a decompression condition for the predetermined period (for example, in the case where a subject plant is mouse-ear cress, it may be placed at approximately 400 mm Hg (approximately 50 kPa) for 4-12 minutes), and thereafter, the decompression condition is gradually released. After the treatment, the plant body is subjected to a suitable acclimatization condition to grow, and the transformant is selected on a medium containing an antibiotic according to the conventional method, and optionally a plant seed may be obtained from the transformant.

### Example

### Transformation of mouse-ear cress (Arabidopsis thaliana)

A cylindrical microporous body for early cultivation made from Murakami clay (collected in Niigata Prefecture, Japan) (outer diameter 14 mm, inner diameter 9 mm, height 45 mm) was immersed in the first aqueous fertilizer (0.1% of Hyponex), and a mouse-ear cress seed was placed on an upper end surface of a wet microporous body.

After four weeks cultivation under continuous lumination at 3000 lux at 23 °C, a grown plant body was repotted to a cylindrical microporous body for expanded cultivation made from the same material (outer diameter 14 mm, inner diameter 9 mm, height 85 mm) immersed in the second aqueous fertilizer (NaH₂PO₄? 2H₂O 1.5 mM, Na₂HPO₄? 12H₂O 0.25 mM, MgSO₄? 7H₂O 1.5 mM, Ca(NO₃)₂? 4H₂O 2 mM, KNO₃ 3 mM, Na₂? EDTA 67 µM, FeSO₄? 7H₂O 8.6 µM, MnSO₄? 4H₂O 10.3 µM, H₃BO₃ 30 µM, ZnSO₄? 7H₂O 1.0 µM, CuSO₄? 5H₂O 1.0 µM, (NH₄)₆? Mo₇O₂₄? 4H₂O 0.024 µM, CoCl₂? 6H₂O 0.13 µM) such that a root of the plant body was contacted with a wet surface of the microporous body and, thereafter, cultivated under a continuous lumination at 3000 lux at 23 °C for four weeks again.

On the other hand, Agrobacterium tumefacience EHA105 harboring a binary vector pBI121 was incubated in a LB medium containing 10 mg/l of kanamycin at 28 °C for 48 hours. After incubation, a culture was centrifuged, and the resulting bacterial pellet was resuspended in an infiltration medium (IM) of the volume twice of an initial medium (I M=1/2 Gamborg's B-5 medium containing 0.1% Hyponex 5-10-5, 1% sucrose, 0.02% Silwet L-77 and 0.044 µM 6-benzylaminopurine).

The mouse-ear cress plant bodies grown on ten microporous bodies as described above were inverted together, and then immersed in 300 ml of IM medium containing Agrobacterium which was placed in 500 ml volume beaker. Such the mouse-ear cress plant bodies immersed in the beaker were subjected to decompression in a vacuum chamber for 5 minutes. Infiltrated plant bodies were grown for six weeks under the condition as described above, and approximately 10000 seeds were harvested therefrom.

Among the harvested seeds, approximately 5000 seeds were placed on a selective medium (1/2 MS medium containing 100 mg/l kanamycin), and transformed plant bodies which had survived even after 2 weeks were selected.

As the result, transformed plant bodies which correspond to 0.14 %, 0.10 % and 0.12 % of seeds were obtained (triplicate results). On the other hand, it is reported that, in a mouse-ear cress experiment of soil cultivation, 70 transformed plant bodies were obtained from 60000 seeds (0.12 % transformation efficiency, Bechtold et al., cited above). In light of this, it is found that the plant cultivated on the microporous body of the present invention has a similar transformation efficiency to that of a plant cultivated in the soil.

However, as described above, a larger number of plant bodies can be cultivated in a limited area such as in an artificial weather conditioner by the microporous body of the present invention than in a conventional soil, because an area necessary for cultivating one plant body on the microporous body of the present invention can be reduced than in the conventional soil cultivation (in the case of mouse-ear cress; 54 plant bodies/30×40 cm in the soil cultivation, 63 plant bodies/46×2 cm in the microporous body cultivation). Therefore, an effect greater than an actual transformation efficiency is exhibited because a transformation treatment can be conducted to a larger number of plant bodies.

According to the present invention, there can be provided an equipment for transforming plants, a system for transforming plants, and a method for transforming plants, which can be utilized in more exact, convenient, speedy and efficient experiment, investigation and development, and which can supply a healthy and high quality plant in a qualitatively and quantitatively stable manner.

## Claims

1. An equipment for transforming plants which comprises:
a microporous body having a surface on which a plant seed is germinated and grown into a plant body, wherein the plant seed is germinated and grown by absorbing an aqueous nutrition which is retained in communicating pores in the microporous body from the surface of the microporous body; and
a carrier solution containing a gene with which the plant body is transformed,
wherein the grown plant body is transformed by immersing it in the carrier solution according to an in planta method.

2. The equipment for transforming plants according to claim 1, wherein the in plant method is a vacuum infiltration transformation.

3. A system for transforming plants which comprises:
a plurality of microporous bodies, each microporous body having a surface on which a plant seed is germinated and grown into a plant body; and
a holding means for removably holding the plurality of microporous bodies,
wherein each plant seed is germinated and grown by absorbing an aqueous nutrition which is retained in communicating pores in the microporous body from the surface of the microporous body,
wherein a plurality of plant bodies grown on the surfaces of the microporous bodies held by the holding means are transformed by immersing them in a carrier solution approximately at the same time according to an in planta method.

4. The system for transforming plants according to claim 3, wherein the in planta method is a vacuum infiltration transformation.

5. The system for transforming plants according to claim 3 or 4, wherein the aqueous nutrition is stored in a holding means with contacting with the microporous bodies.

6. The system for transforming plants according to claim 3 or 4, which further comprises a storage tank for storing the aqueous nutrition and an aqueous nutrition-supplying means for connecting the microporous body with the aqueous nutrition in the storage tank, wherein the aqueous nutrition in the storage tank is supplied to the microporous body through the aqueous nutrition-supplying means.

7. The system for transforming plants according to any one of claims 3 to 6, wherein the microporous body has a cylindrical shape, and the plant seed is germinated and grown on an inner surface of the microporous body.

8. The system for transforming plants according to any one of claims 3 to 7, wherein the plants are selected from the group consisting of a useful tree such as bishop's flower (Ammi majus), onion (Allium cepa), garlic (Allium sativum), celery (Apium graveolens), asparagus (Asparagus officinalis), sugar beet (Beta vulgaris), cauliflower (Brassica oleracea var. botrytis), brusseles sprout (Brassica oleracea var. gemmifera), cabbage (Brassica oleracea var. capitata), rape (Brassica napus), caraway (Carum carvi), chrysanthemum (Chrysanthemum morifolium), spotted hemlock (Conium maculatum), coptis Rhizome (Coptis japonica), chicory (Cichorium intybus), summer squash (Curcurbita pepo), thorn apple (Datura meteloides), carrot (Daucus carota), carnation (Dianthus caryophyllus), buckwheat (Fagopyrum esculentum), fennel (Foeniculum vulgare), strawberry (Fragaria chiloensis), soybean (Glycine max), hyacinth (Hyacinthus orientalis), sweet potato (Ipomoea batatas), lettuce (Lactuca sativa), birds-foot trefoil (Lotus corniculatus, Lotus japonicus), tomato (Lycopersicon esculentum), alfalfa (Medicago sativa), tobacco (Nicotiana tabacum), rice (Oryza sativa), parsley (Petroselinum hortense), pea (Pisum sativum), rose (Rosa hybrida), egg plant (Solanum melongena), potato (Solanum tuberosum), wheat (Triticum aestivum), maize (Zea mays), sugar beat(Beta vulgaris), cotton (Gossypium indicum), rape (Brassica campestris), flax (Linum usitatissimum), sugarcane (Saccharum officinarum), papaya (Carica papaya), Squash (Cucurbita moschata), cucumber (Cucumis sativus), watermelon (Citrullus vulgaris), melon (Cucumis melo), Winter Squash (Cucurbita maxima) and the like; a foliage plant such as snapdragon (Antirrhinum majus), mouse-ear cress (Arabidopsis thaliana), croton (Codiaeum variegatum), cyclamen (Cyclamen persicum), poinsettia (Euphorbia pulcherrima), barberton daisy (Gerberajamesonii), sunflower (Helianthus annuus), fish geranium (Pelargonium hortorum), petunia (Petunia hybrida), African violet (Saintpaulia ionatha), dandelion (Taraxacum officinale), torenia (Torenia fournieri), Dutch clover (Trifolium repens), cymbidium (Cymbidium) and the like; a woody plant such as beat tree (Azadirachta indica), orange (Citrus), common coffee (Coffea arabica), ribbon gum (Eucalyptus), para rubber tree (Hevea brasiliensis), holly (Ilex aquifolium), trifoliate orange (Poncirus trifoliata), almond (Prunus amygdalus), carolina poplar (Populus canadensis), oriental arborvitae (Biota orientalis), Japanese ceder (Cryptomeriajaponica), Norway spruce (Picea abies), pine genus (Pinus), grapevine (Vitis vinifera), apple (Malus pumila), apricot (Prunus armeniaca), persimmon (Diospyros kaki), fig (Ficus carica), chestnut (Castanea crenata), Lombardy poplar(Populus nigra), Eleuthero(Acanthopanax senticosus) and the like.

9. A method for transforming plants which comprises steps of:
germinating and growing a plant seed into a plant body on a surface of a microporous body, wherein the plant seed is grown by absorbing an aqueous nutrition retained in communicating pores in the microporous body from the surface of the microporous body; and
transforming the plant body grown on the surface of the microporous body by immersing it in a carrier solution containing a gene with which the plant body is transformed according to an in planta method.

10. The method for transforming plants according to claim 9, wherein the in planta method is a vacuum infiltration transformation.

11. A method for transforming plants which comprises steps of:
removably holding a plurality of microporous bodies in a holding means;
seeding a plant seed on each surface of the microporous bodies, wherein the plant seed is germinated and grown into a plant body by absorbing an aqueous nutrition retained in communicating pores in the microporous body from the surface of the microporous body; and
transforming a plurality of plant bodies grown on the surfaces of a plurality of the microporous bodies held in the holding means by immersing them in a carrier solution containing a gene with which the plant body is transformed approximately at the same time according to an in planta method.

12. The method for transforming plants according to claim 11, wherein the in planta method is a vacuum infiltration transformation.

13. The method for transforming plants according to claim 11 or 12, which further comprises a step of selecting only microporous bodies having the plant bodies which have grown to a stage suitable for transformation to hold them in the holding means before immersing the plant bodies in the carrier solution, and subjecting the plant bodies to transformation.

14. A method for selecting plants harboring a heterogeneous gene from a parent transformed plant body, which comprises steps of:
(i) immersing a portion of a microporous body in an aqueous nutrition containing one or more of the first drug for selection;
(ii) seeding a plant seed obtained from a transformed plant body on a surface of the microporous body, wherein the transformed plant body has been transformed with at least one heterogeneous gene comprising a resistant gene for the first drug for selection and wherein the plant seed harboring the heterogeneous gene from a parent transformed plant body can be germinated or grown by absorbing an aqueous nutrition containing the first drug for selection retained in communicating pores in the microporous body from the surface of the microporous body, but the plant seed harboring no heterogeneous gene from a parent transformed plant body can not be germinated or grown; and
(iii) obtaining the plant body which can be germinated or grown or, further, repeating above steps (i) to (iii) once or more times, using the plant seed obtained from the plant body and one or more of the drug for selection which is the same as or different from the first drug for selection in place thereof, wherein the transformed plant body also comprises a resistant gene for the drug for selection.

15. A method for selecting plants harboring a heterogeneous gene from a parent transformed plant body which comprises conducting, at least one time, steps of:
seeding a plant seed obtained in claim 14 harboring the resistant gene for the first drug for selection on the surface of the microporous body, wherein the plant seed is germinated and grown into a plant body by absorbing one or more of a drug for selection different from the first drug for selection or the aqueous nutrition retained in communicating pores in the microporous body from the surface of the microporous body; and
confirming whether the grown plant body harbors the resistant gene for the drug for selection different from the first drug for selection, or whether the grown plant body expresses a target heterogeneous gene as phenotype thereof.
